# EUROPEAN PATENT SPECIFICATION

(11) **EP 3 185 973 B1**
(45) Date of publication and mention of the grant of the patent: **25.11.2020**
(21) Application number: 15829629.3
(22) Date of filing: 30.07.2015
(51) Int. Cl.: A62D 3/02, A62D 3/20, A62D 3/37

(54) **USE OF A SULFIDE QUINONE REDUCTASE ENZYME FOR SCAVENGING HYDROGEN SULFIDE AND/OR MERCAPTANS**
VERWENDUNG EINES SULFID-CHINON-REDUKTASE-ENZYMS ZUM ABFANGEN VON WASSERSTOFFSULFID UND / ODER MERCAPTANEN
UTILISATION D'UNE ENZYME DE SULFURE QUINONE REDUCTASE POUR LE NETTOYAGE DE SULFURE D'HYDROGENE ET / OU DE MERCAPTANS

(30) Priority: 07.08.2014 US 201414454417
(43) Date of publication of application: 05.07.2017
(73) Proprietor: Baker Hughes Holdings LLC, Houston, TX 77073 (US)
(72) Inventor: DHULIPALA, Prasad D., Katy TX 77494 (US); ARMSTRONG, Charles David, Tomball, Texas 77377 (US); QU, Qi, Spring, Texas 77379 (US)
(74) Representative: BRP Renaud & Partner mbB Rechtsanwälte Patentanwälte Steuerberater
(86) International application number: PCT/US2015/042794
(87) International publication number: WO 2016/022367

(56) References cited:
- WO-A1-2012/166964
- US-A1- 2008 190 844
- US-A1- 2009 181 081
- US-A1- 2013 273 277
- US-B1- 6 306 288
- Christoph Griesbeck ET AL: "Biological Sulfide Oxidation: Sulfide-Quinone Reductase (SQR), the Primary Reaction", Recent Research Developments in Microbiology Research Signpost Recent Research Developments in Microbiology Research Signpost, 1 January 2000 (2000-01-01), pages 179-203, XP55452057, Retrieved from the Internet: URL:http://www.biologie.uni-regensburg.de/ Botanik/Hauska/sqr-review.pdf
- DATABASE GENBANK [Online] 13 July 2011 'SULFIDE-QUINONE REDUCTASE, PUTATIVE, PARTIAL (ACIDITHIOBACILLUS SP.GGI-221)', XP055395692 Retrieved from NCBI Database accession no. EGQ63573.1
- BRASSEUR, G. ET AL.: 'APPARENT REDUNDANCY OF ELECTRON TRANSFER PATHWAYS VIA BC"1 COMPLEXES AND TERMINAL OXIDASES IN THE EXTREMOPHILIC CHEMOLITHOAUTOTROPHICACIDITHIOBACILLUSFERRO OXIDANS' BIOCHIMICA ET BIOPHYSICA ACTA vol. 1656, no. 2-3, 07 June 2004, pages 114 - 126, XP004514818 DOI: 10.1016/J.BBABIO.2004.02.008

## Description

### Field of the Disclosure

The disclosure relates to use of a sulfide quinone reductase enzyme, having the amino acid sequence set forth in SEQ ID 3, for scavenging hydrogen sulfide and/or mercaptans from a liquid or gaseous stream.

### Background of the Disclosure

Hydrogen sulfide and mercaptans are present in underground water removed with crude oil, in crude oil itself, in natural gases and in gases associated with underground water and crude oil. Hydrogen sulfide and mercaptans are characterized by highly noxious odors and typically are highly corrosive. Uncontrolled emissions of hydrogen sulfide give rise to severe health hazards. The presence of hydrogen sulfide and mercaptans is further objectionable because they often react with desirable hydrocarbons as well as fuel system components.

Treatments for removal of hydrogen sulfide and mercaptans from hydrocarbons and other substrates include the use of various reactive organic compounds as scavengers. For example, U.S. Patent No. 6,063,346 discloses the use of maleimides, formaldehydes, amines, carboxamides, alkylcarboxyl-azo compounds and cumine-peroxide compounds for the removal of hydrogen sulfide and mercaptans. Further, U.S. Patent No. 5,128,049 discloses the use of certain morpholino and amino derivatives for the removal of hydrogen sulfide. In addition, U.S. Patent Nos. 8,022,017; 7,264,786; 6,063,346 and 5,128,049 disclose the use of triazines to remove hydrogen sulfide. Such scavengers however are often considered to be undesirable since they are not environmentally preferable. US2008/190844 discloses a method to scavenge hydrogen-sulfide or mercaptons from a gas or liquid using Thiobacillus ferrooxidans.

Since the generation of hydrogen sulfide and mercaptans is often encountered throughout drilling, production, transport, storage and processing of crude oil as well as underground water, there is a need for use of an enzyme for scavenging the formation of hydrogen sulfide and mercaptans and the deleterious effects created by hydrogen sulfide and mercaptans in an environmentally preferable manner.

Accordingly, there exists a need for an environmentally friendly or "green" use of an enzyme for scavenging hydrogen sulfide and/or mercaptans within a liquid or gaseous stream having one or more of the attributes or capabilities described or shown in, or as may be apparent from, the other portions of this patent.

### Summary of the Disclosure

The present invention provides a use of a sulfide quinone reductase enzyme having the amino acid sequence as set forth in SEQ ID 3 for scavenging hydrogen sulfide and/or mercaptans from a liquid or gaseous stream as claimed in claim 1.

The liquid or gaseous stream may be within a hydrocarbon producing reservoir.

The liquid or gaseous stream may be within a storage vessel.

The liquid or gaseous stream may be within an unrefined or refined hydrocarbon product derived from petroleum or from the liquefaction of coal.

In an embodiment, a liquid or gaseous stream is brought into contact with a SQR enzyme. Hydrogen sulfide and/or mercaptans are then oxidized to sulfur containing oxidation product.

Characteristics and advantages of the present disclosure described above and additional features and benefits will be readily apparent to those skilled in the art upon consideration of the following detailed description of various embodiments and referring to the accompanying figures.

### Brief Description of the Drawings

The following figures are part of the present specification, included to demonstrate certain aspects of various embodiments of this disclosure and are referenced in the detailed description herein:
FIG. 1 depicts an alignment (SEQ ID 1) between a known amino acid sequence of sulfide quinone reductase (SQR) enzyme (SEQ ID 2) and the amino acid sequence of the sulfide quinone reductase enzyme (SEQ ID 3) used in the present invention as a hydrogen sulfide scavenger.
FIG. 2 illustrates the effectiveness of a SQR enzyme as a hydrogen sulfide scavenger in soured well water.
FIG. 3 illustrates reduction of H₂S/sulfide by a SQR enzyme from a sodium sulfide solution.
FIG. 4 illustrates the functionality of a SQR enzyme as a hydrogen sulfide barrier.
FIG. 5 demonstrates the effect of pH on the activity of a SQR enzyme on the reduction of hydrogen sulfide.
FIG. 6 demonstrates the effect of temperature on the activity of a SQR enzyme.
FIG. 7 illustrates the tolerance of a SQR enzyme to salt.
FIG. 8 illustrates the reduction of hydrogen sulfide from head space using a SRQ enzyme.

### Detailed Description of the Preferred Embodiments

Characteristics and advantages of the present disclosure and additional features and benefits will be readily apparent to those skilled in the art upon consideration of the following detailed description of exemplary embodiments of the present disclosure. It should be understood that the description herein, being of example embodiments, are not intended to limit the claims of this patent or any patent or patent application claiming priority hereto. Many changes may be made to the particular embodiments and details disclosed herein without departing the scope of the appended claims.

As used herein and throughout various portions (and headings) of this patent application, the terms "disclosure", "present disclosure" and variations thereof are not intended to mean every possible embodiment encompassed by this disclosure or any particular claim(s). Thus, the subject matter of each such reference should not be considered as necessary for, or part of, every embodiment hereof or of any particular claim(s) merely because of such reference. Also, the terms "including" and "comprising" are used herein and in the appended claims in an open-ended fashion, and thus should be interpreted to mean "including, but not limited to ...."

In the present disclosure, a liquid or gaseous stream rendered "sour" by the presence of sulfhydryl compounds is brought into contact with a catalytically effective amount of a sulfide quinone reductase (SQR) enzyme. The liquid or gaseous stream may be an aqueous substrate or a hydrocarbon substrate.

As used herein, the term "mercaptan" shall include alkyl mercaptans and thiols of the formula R-SH where R is an unsubstituted or substituted alkyl, thiol carboxylic acids and dithio acids.

As used herein, the term "aqueous substrate" shall refer to any "sour" aqueous substrate, including waste water streams in transit to or from municipal waste water treatment facilities, tanning facilities, and the like. The term "hydrocarbon substrate" is meant to include unrefined and refined hydrocarbon products, including natural gas, derived from petroleum or from the liquefaction of coal, both of which contain hydrogen sulfide or other sulfur-containing compounds. Thus, particularly for petroleum-based fuels, the term "hydrocarbon substrate" includes, but is not limited to, wellhead condensate as well as crude oil which may be contained in storage facilities at the producing field. "Hydrocarbon substrate" also includes the same materials transported from those facilities by barges, pipelines, tankers, or trucks to refinery storage tanks, or, alternately, transported directly from the producing facilities through pipelines to the refinery storage tanks. The term "hydrocarbon substrate" also includes refined products, interim and final, produced in a refinery, including distillates such as gasoline, distillate fuels, oils, and residual fuels and to vapors produced by the foregoing materials. The use defined herein is therefore applicable to a wide variety of fluid streams, including liquefied petroleum gas as well as crude oil and petroleum residual fuel, heating oil, etc. In addition, the method is applicable to gaseous hydrocarbon streams. For instance, the composite may be contacted with wet or dry gaseous mixtures of hydrogen sulfide and/or mercaptan and hydrocarbon vapors, such as is found, for instance, in natural gas or obtained in the drilling, removal from the ground, storage, transport, and processing of crude oil.

The use disclosed herein has particular applicability in the removal of hydrogen sulfide and mercaptans of the formula R-SH wherein R is an alkyl group having from 1 to 40 carbon atoms and preferably from 1 to 20 carbon atoms, most preferably from 1 to 6 carbon. Such mercaptans are especially desirable for removal in light of their noxious odors and corrosive nature.

The sulfide quinone reductase (SQR) enzyme used as a hydrogen sulfide scavenger disclosed herein may originate from various organisms. The SQR enzyme prevents the formation of hydrogen sulfide and mercaptans. The harm from such materials may be seen within the reservoir, in transport pipes as well as in storage areas and containers for fluids produced from the reservoir. In addition to contributing to corrosion of metals used during production, transport and/or storage of produced fluids, the presence of hydrogen sulfide and mercaptans further causes reservoir souring and presents a danger to oilfield personnel.

The dangers of hydrogen sulfide and mercaptans are mitigated by the addition of the SQR enzyme to the liquid or gaseous stream. The addition of SQR enzyme to the liquid or gaseous stream is believed to catalytically attack hydrogen sulfide and precursors of hydrogen sulfide. It further prevents the formation of hydrogen sulfide and mercaptans. It is believed that the SQR enzyme catalyzes the oxidative breakdown of hydrogen sulfide and mercaptans to sulfur containing oxidation product. The oxidation product could be elemental sulfur, a sulfite, a polysulfide, etc.

The SQR enzyme may remain stable and be effective at temperatures in excess of 96°C (205°F). Typically, the SQR enzyme is introduced into a liquid or gaseous substrate maintained at a temperature between from about -4 to about 82°C (25 to about 180°F), more typically between from about 38 to about 66°C (100 to about 150°F).

The amount of SQR enzyme added to the liquid or gaseous stream is that amount sufficient to effectuate the desired result over a sustained period of time and thus is dependent on the amount of the hydrogen sulfide and/or mercaptan in the medium being treated. In general, the amount of the SQR enzyme added to the medium is at least an effective scavenging amount, for example, from about 0.05 ppm to about 2,000 ppm or more, preferably from about 20 to about 1,200 ppm, and more preferably from about 100 to about 400 ppm of hydrogen sulfide and/or mercaptan.

The SQR enzyme is particularly effective in the treatment of liquid or gaseous streams having a pH between from about 5.5 to about 7.5.

Since SQR catalytically breaks down hydrogen sulfide and mercaptans, it may be used over an extended period of time (until it is no longer stable) without being replenished. In addition, the use of SQR inflicts reduced, minimal, or no harm to an aqueous or hydrocarbon substrate.

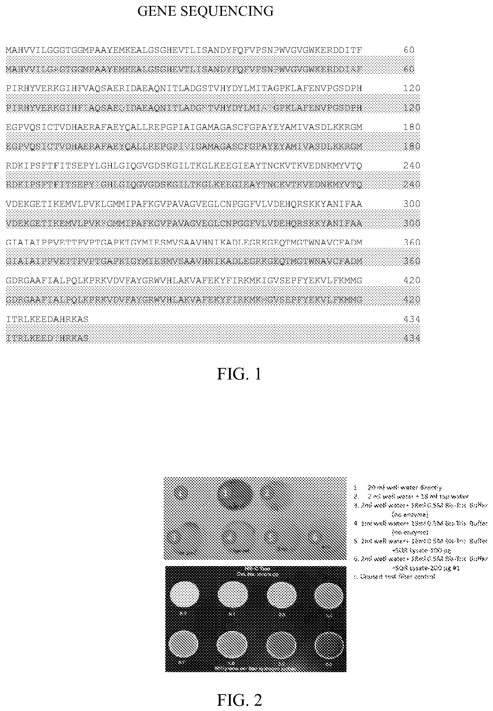

The nucleotide sequence encoding the SQR enzyme may be derived from a gram negative, acidophilic and thermophilic bacterium, such as *Acidithiobacillus ferrooxidans, Metallospora cuprina* and *Metallospora sedula,* using polymerase chain reaction (PCR) amplification. The amino acid sequence of SQR reductase enzyme used in this invention is set forth in SEQ ID 3. SQR variants of *Acidithiobacillus ferrooxidans* is set forth in FIG. 1. The SQR gene sequence was amplified using *A. ferrooxidans* genomic DNA and was cloned in a protein expression vector. A homology may be similar for other SQR enzymes depending on originating organisms.

The SQR enzyme may be added to any aqueous or nonaqueous medium containing hydrogen sulfide and/or mercaptans where sulfides are sought to be mitigated. Such media include wet gaseous mediums containing water vapors and/or hydrocarbon vapors. Thus, the method disclosed herein is useful in controlling hydrogen sulfide and/or mercaptans in water systems, oil and gas production and storage systems, and other similar systems.

Generally, for industrial or commercial use, the SQR enzyme may be contacted with a stream containing the hydrogen sulfide or mercaptans for removal. Contact can occur in a variety of containers, such as a process or transport line, a separate stirred or non-stirred container or other vessels such as scrubbers or strippers. Further, the SQR enzyme may be added via surface or downhole equipment or at any time in the process stream in recovering crude oil so as to remove the noxious quality and corrosive nature of the hydrogen sulfide and mercaptans in the processing system.

In general, the SQR enzyme is injected into or otherwise brought into intimate contact with the liquid hydrocarbon, hydrogen sulfide and/or mercaptan and, when present, water and/or solvent in any convenient manner. The SQR enzyme has particular usefulness where the liquid or gaseous stream being treated is sour well water.

With emissions from a residual fuel oil, the SQR enzyme may be stirred into the fuel oil. When used with a natural gas, the natural gas may be scrubbed with an aqueous or nonaqueous solution containing the SQR enzyme. Additionally, when the natural gas, as it often does, contains water vapors, the SQR enzyme may be injected into a stream of the gas moving within a conduit. In such case, when the water vapors are removed from the natural gas as a liquid, the product resulting from the catalytic reaction of the hydrogen sulfide may then be removed.

In another embodiment, the liquid or gaseous stream being treated with the SQR enzyme may be sea water or brine. The liquid or gaseous stream being treated may be within a hydrocarbon producing reservoir or within a storage vessel. Further, the liquid or gaseous stream may be an unrefined or refined hydrocarbon product derived from petroleum or from the liquefaction of coal. Further, the liquid or gaseous stream may be a wet or dry gaseous mixture or hydrocarbon vapors.

The SQR enzyme may be added neat or diluted with water or solvent and may be formulated or blended with other suitable materials or additives. In a preferred embodiment, the SQR enzyme is included in a brine (such as a saturated potassium chloride or sodium chloride solution), salt water or fresh water. The diluent is selected in order for the SQR enzyme to be soluble both in the diluents and in the feed stream. For liquid systems, suitable solvents for dissolving the scavenger include polar and non-polar solvents such as water, alcohols, esters, benzene and benzene derivatives. The SQR enzyme may further be advantageously employed in liquefied gas and foamed gas carrier fluids, such as liquid CO₂, CO₂/N₂, and foamed N₂ in CO₂ based systems.

Well treatment compositions containing the SQR enzyme may further be gelled or non-gelled. Suitable carrier fluids include or may be used in combination with fluids have gelling agents, cross-linking agents, gel breakers, surfactants, foaming agents, demulsifiers, buffers, clay stabilizers, acids, or mixtures thereof. The SQR enzyme may be used in any well treatment operation where the presence of hydrogen sulfide and/or mercaptans may be encountered. As such, the SQR enzyme may be a component of a fracturing fluid (with or without the presence of a proppant), an acidizing fluid, drilling fluid, completion fluid, etc. In addition, the SQR enzyme may be used during the transport, storage and/or processing of oil or gas to address issues raised by the presence of hydrogen sulfide and/or mercaptans.

Preferred embodiments of the present disclosure thus offer advantages over the prior art and are well adapted to carry out one or more of the objects of this disclosure. However, the present disclosure does not require each of the components and acts described above and are in no way limited to the above-described embodiments or methods of operation. Any one or more of the above components, features and processes may be employed in any suitable configuration without inclusion of other such components, features and processes. The invention is defined in its broadest sense by the appended claims.

All percentages set forth in the Examples are given in terms of weight units except as may otherwise be indicated.

The SQR enzyme used in the Examples had the amino acid sequence set forth in FIG. 1 and was prepared as discussed in the paragraphs above.

### EXAMPLES

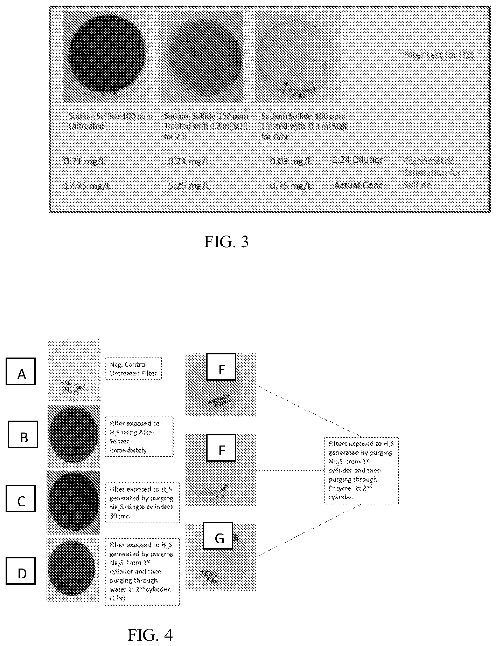

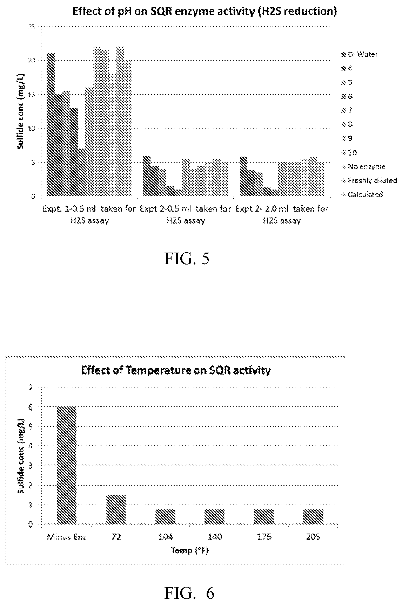

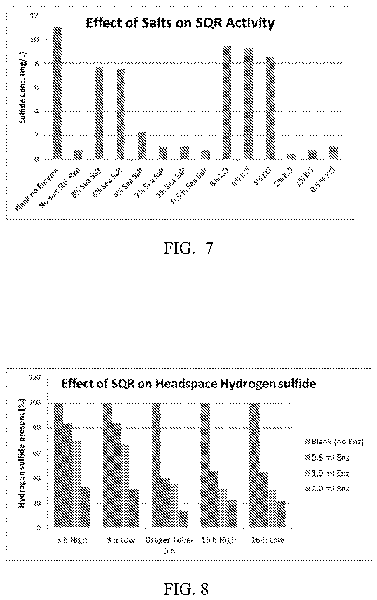

Example 1. This example illustrates the reduction of hydrogen sulfide in enzyme treated samples. Referring to FIG. 2, 20 ml of sour well water directly or the well water diluted 10 times using tap water (2) or 0.05M Bis-tris buffer (3) or well water diluted 20 times with 0.05 M Bis-Tris buffer (4) was used to measure hydrogen sulfide concentration. 100 µg or 200 µg of SQR crude lysate was added to the samples diluted 10 times with 0.05 M Bis-tris buffer and hydrogen sulfide measurements were performed after 30 min incubation to evaluate enzyme performance. A comparison of the results with a reference card supplied with the HACH filter assay kit revealed reduction of hydrogen sulfide in enzyme treated samples (5 and 6).
Example 2. This example illustrates the reduction of H₂S sulfide by a SQR enzyme from a sodium sulfide solution. Referring to FIG. 3, sodium sulfide solution was used as the standard to generate hydrogen sulfide to test the SQR enzyme. 100 ppm of sulfide solution was prepared from sodium sulfide·9H₂0 in water and equal amount of 0.1 M Bis-Tris buffer, pH 6 was added. 100 ml of this solution was incubated with 1 ml of SQR enzyme lysate for 2 h or 18 h. Sulfide concentrations were measured by HACH filter assay or by HACH colorimetric method. The results in FIG. 3 show that treatment of sulfide solution with SQR reduced sulfide to 30% in 2 hours and to 6% in 18 hours.
Example 3. This Example illustrates the use of the SQR enzyme as a barrier to hydrogen sulfide. Referring to FIG. 4, an unreacted filter was used as negative control (A) and 100 ppm Na₂S solution was taken in to a bottle and exposed to filter (B), 150 ml of 100 ppm Na₂S solution was taken in a cylinder and purged with air. The air from the other end of the cylinder was collected on to filter paper (C). 150 ml of 100 ppm Na₂S solution was taken in a first cylinder and purged with air. The air from the other end of the cylinder was purged through water in the second cylinder and the air from the other end of the 2^{nd} cylinder was collected onto filter paper (D). Both C and D were taken as minus enzyme controls. To evaluate enzyme efficiency, the second cylinder was filled with 100 ml of 0.05M Bis-Tris buffer containing 2 ml of enzyme lysate and air was purged similar to C or D. The air from second cylinder was collected on the filter in 3 independent experiments (E, F, and G). Results show that the filters remained light blue indicating that there is no or very slight hydrogen sulfide in the air coming out from the enzyme cylinder.
Example 4. This Example illustrates the effect of pH on the activity of the SQR enzyme. 0.1 M Bis-tris buffers with pH 4, 5, 6, 7, 8, 9 and 10 were prepared. 100 ppm sodium sulfide solution was prepared in water. To 25 ml of 100 ppm sodium sulfide, 25 ml of 0.1 M Bis-Tris buffer was added and mixed well. To this was added 0.5 ml of SQR enzyme lysate and the reactions were incubated for 18 hours. The sulfide concentrations were measured by HACH colorimetric method. FIG. 5 shows the optimal pH for the SQR enzyme to be at 6 and 7 where it reduced the maximum amount of sulfide.
Example 5. This Example illustrates the resistance to high temperature of the SQR enzyme. 100 ppm sodium sulfide solution was prepared in water. To 25 ml of 100 ppm sodium sulfide, 25 ml of 0.1 M Bis-Tris buffer was added and mixed well. To this 0.5 ml of SQR enzyme lysate was added and the reactions were incubated for 18 hours at different temperatures. The sulfide concentrations were measured by HACH colorimetric method. The results set forth in FIG. 6 show the SQR enzyme to exhibit good activity at all temperatures tested and that sulfide was reduced 12.5% from 40°C to 96°C (104°F to 205°F).
Example 6. This Example illustrates the tolerance of the SQR enzyme to salt concentrations present in brine. 100 ppm sodium sulfide solution was prepared in water containing 1% to 16% sea salt or potassium chloride. To 25 ml of 100 ppm sodium sulfide containing salts, 25 ml of 0.1 M Bis-Tris buffer was added and mixed well. To this 0.5 ml of SQR enzyme lysate was added and the reactions were incubated for 18 hours at different temperatures. The sulfide concentrations were measured by HACH colorimetric method. The results illustrated in FIG. 7 show that the SQR enzyme had tolerance to up to 4% sea salt and 2% potassium chloride.
Example 7. This Example illustrates reduction of hydrogen sulfide gas with the SQR enzyme from head space. 100 ppm sodium sulfide solution was prepared in water containing in 0.05 M Bis Tris buffer, pH 7.0. In eight 200 ml injection bottles, 90 ml of 100 ppm sulfide solution was taken and sealed airtight with septum. The bottles were divided into 2 sets of 4. First set bottles 1-4 were allowed to stand for 1.5 hours to reach equilibrium of hydrogen sulfide in head space. Initial hydrogen sulfide concentrations were measured by a Micro Gas Chromatography method. To bottle 1, no enzyme was added. To bottle 2 0.5 ml, to bottle 3-1ml and to bottle 4 two ml enzyme lysates were added. The reactions were incubated for 16 hours. The sulfide concentrations were measured by Micro GC. Similarly second set of bottles 5-8 were allowed to stand for 16 hours to reach equilibrium of hydrogen sulfide in head space. Initial hydrogen sulfide concentrations were measured by a Micro Gas Chromatography method. To bottle 5, no enzyme was added. To bottle 6-0.5 ml, to bottle 7-1ml and to bottle 8-2 ml enzyme lysates were added. The reactions were incubated for 3 hours. The sulfide concentrations were measured by Micro GC. Low and High in FIG. 8 represent the two columns with low and high sensitivities that give accurate hydrogen sulfide concentrations. The concentration of hydrogen sulfide was also measured by Dragger tubes which confirmed the Micro GC data. and methods of the present disclosure, such as in the components, details of construction and operation, arrangement of parts and/or methods of use, are possible, contemplated by the patent applicant(s), within the scope of the appended claims, and may be made and used by one of ordinary skill in the art without departing from the spirit or teachings of the disclosure and scope of appended claims. Thus, all matter herein set forth or shown in the accompanying drawings should be interpreted as illustrative, and the scope of the disclosure and the appended claims should not be limited to the embodiments described and shown herein.

## Claims

1. Use of a sulfide quinone reductase enzyme, having the amino acid sequence set forth in SEQ ID 3, for scavenging hydrogen sulfide and/or mercaptans from a liquid or gaseous stream which comprises;
bringing the liquid or gaseous stream into contact with a catalytically effective amount of the sulfide quinone reductase enzyme; and
scavenging the hydrogen sulfide and/or mercaptans in the liquid or gaseous stream with the sulfide quinone reductase enzyme.

2. The use of claim 1, wherein the nucleotide sequence encoding the sulfide quinone reductase enzyme is derived from *Acidithiobacillus ferrooxidans.*

3. The use of claim 1, wherein the liquid or gaseous stream is sour well water.

4. The use of claim 1, wherein the liquid or gaseous stream is sea water or brine.

5. The use of claim 1, wherein the liquid or gaseous stream has a pH between from 5.5 to 7.5.

6. The use of claim 1, wherein the sulfide quinone reductase enzyme is stable and effective at a temperature up to 96°C (205°F).

7. The use of claim 1, wherein the liquid or gaseous stream is within a hydrocarbon producing reservoir.

8. The use of claim 1, wherein the liquid or gaseous stream is within an unrefined or refined hydrocarbon product derived from petroleum or from the liquefaction of coal.

9. The use of claim 1, wherein the liquid or gaseous stream is a wet or dry gaseous mixture or hydrocarbon vapors.

10. The use of claim 1, wherein the liquid or gaseous stream is within a storage vessel.

## Patentansprüche

1. Verwendung eines Sulfid-Chinon-Reduktase-Enzyms, das eine in SEQ ID 3 aufgeführten Aminosäuresequenz zum Abfangen von Wasserstoffsulfid und/oder Mercaptanen aus einem flüssigen oder gasförmigen Strom aufweist, umfassend;
Inkontaktbringen des flüssigen oder gasförmigen Stromes mit einer katalytisch wirksamen Menge des Sulfid-Chinon-Reduktase-Enzyms; und
Abfangen des Wasserstoffsulfids und/oder der Mercaptane in dem flüssigen oder gasförmigen Strom mit dem Sulfid-Chinon-Reduktase-Enzym.

2. Verwendung nach Anspruch 1, wobei die Nukleotidsequenz, die das Sulfid-Chinon-Reduktase-Enzym codiert, aus *Acidithiobacillus ferrooxidans* abgeleitet ist.

3. Verwendung nach Anspruch 1, wobei der flüssige oder gasförmige Strom saures Quellwasser ist.

4. Verwendung nach Anspruch 1, wobei der flüssige oder gasförmige Strom Meerwasser oder Sole ist.

5. Verwendung nach Anspruch 1, wobei der flüssige oder gasförmige Strom einen pH-Wert zwischen 5,5 und 7,5 hat.

6. Verwendung nach Anspruch 1, wobei das Sulfid-Chinon-Reduktase-Enzym stabil und wirksam bei einer Temperatur von bis zu 96 °C (205 °F) ist.

7. Verwendung nach Anspruch 1, wobei sich der flüssige oder gasförmige Strom in einer Kohlenwasserstoff produzierenden Lagerstätte befindet.

8. Verwendung nach Anspruch 1, wobei sich der flüssige oder gasförmige Strom in einem nicht raffinierten oder raffinierten Kohlenwasserstoffprodukt befindet, der aus Erdöl oder aus der Verflüssigung von Kohle abgeleitet ist.

9. Verwendung nach Anspruch 1, wobei der flüssige oder gasförmige Strom ein nasses oder trockenes gasförmiges Gemisch oder Kohlenwasserstoffdampf ist.

10. Verwendung nach Anspruch 1, wobei sich der flüssige oder gasförmige Strom in einem Lagerbehälter befindet.

## Revendications

1. Utilisation d'une enzyme sulfure quinone-réductase, présentant la séquence d'acides aminés présentée dans SEQ ID 3, pour la fixation de sulfure d'hydrogène et/ou de mercaptans à partir d'un courant liquide ou gazeux qui comprend :
la mise en contact du courant liquide ou gazeux avec une quantité catalytiquement efficace de l'enzyme sulfure quinone-réductase ; et
la fixation du sulfure d'hydrogène et/ou des mercaptans dans le courant liquide ou gazeux avec l'enzyme sulfure quinone-réductase.

2. Utilisation selon la revendication 1, dans laquelle la séquence nucléotidique codant pour l'enzyme sulfure quinone-réductase est dérivée *d'Acidithiobacillus ferrooxidans.*

3. Utilisation selon la revendication 1, dans laquelle le courant liquide ou gazeux est de l'eau de puits acide.

4. Utilisation selon la revendication 1, dans laquelle le courant liquide ou gazeux est de l'eau de mer ou de la saumure.

5. Utilisation selon la revendication 1, dans laquelle le courant liquide ou gazeux présente un pH compris entre 5,5 et 7,5.

6. Utilisation selon la revendication 1, dans laquelle l'enzyme sulfure quinone-réductase est stable et efficace à une température allant jusqu'à 96 °C (205 °F).

7. Utilisation selon la revendication 1, dans laquelle le courant liquide ou gazeux est à l'intérieur d'un réservoir de production d'hydrocarbures.

8. Utilisation selon la revendication 1, dans laquelle le courant liquide ou gazeux est à l'intérieur d'un produit hydrocarbure non raffiné ou raffiné dérivé du pétrole ou de la liquéfaction du charbon.

9. Utilisation selon la revendication 1, dans laquelle le courant liquide ou gazeux est un mélange gazeux humide ou sec ou des vapeurs d'hydrocarbure.

10. Utilisation selon la revendication 1, dans laquelle le courant liquide ou gazeux est à l'intérieur d'un récipient de stockage.


### SEQUENCE LISTING

<110> Baker Hughes Incorporated
<120> METHOD OF MITIGATING HYDROGEN SULFIDE OR MERCAPTAN CONTAMINATION WITH AN ENZYME BASED SCAVENGER
<130> 020569-35100 (55154)
<140> 14/454,417
   <141> 2014-08-07
<160> 1
<170> Patent In version 3.5
<210> 1
   <211> 868
   <212> PRT
   <213> Acidithiobacillus ferrooxidans
<400> 1
   Met Ala His Val Val Ile Leu Gly Gly Gly Thr Gly Gly Met Pro Ala
   1               5                   10                  15
   Ala Tyr Glu Met Lys Glu Ala Leu Gly Ser Gly His Glu Val Thr Leu
               20                  25                  30
   Ile Ser Ala Asn Asp Tyr Phe Gln Phe Val Pro Ser Asn Pro Trp Val
           35                  40                  45
   Gly Val Gly Trp Lys Glu Arg Asp Asp Ile Thr Phe Met Ala His Val
       50                  55                  60
   Val Ile Leu Gly Ala Gly Thr Gly Gly Met Pro Ala Ala Tyr Glu Met
   65                  70                  75                  80
   Lys Glu Ala Leu Gly Ser Gly His Glu Val Thr Leu Ile Ser Ala Asn
                   85                  90                  95
   Asp Tyr Phe Gln Phe Val Pro Ser Asn Pro Trp Val Gly Val Gly Trp
               100                 105                 110
   Lys Glu Arg Asp Asp Ile Ala Phe Pro Ile Arg His Tyr Val Glu Arg
           115                 120                 125
   Lys Gly Ile His Phe Val Ala Gln Ser Ala Glu Arg Ile Asp Ala Glu
       130                 135                 140
   Ala Gln Asn Ile Thr Leu Ala Asp Gly Ser Thr Val His Tyr Asp Tyr
   145                 150                 155                 160
   Leu Met Ile Thr Ala Gly Pro Lys Leu Ala Phe Glu Asn Val Pro Gly
                   165                 170                 175
   Ser Pro His Pro Pro Ile Arg His Tyr Val Glu Arg Lys Gly Ile His
               180                 185                 190
   Phe Ile Ala Gln Ser Ala Glu Gln Ile Asp Ala Glu Ala Gln Asn Ile
           195                 200                 205
   Thr Leu Ala Asp Gly Asn Thr Val His Tyr Asp Tyr Leu Met Ile Ala
       210                 215                 220
   Thr Gly Pro Lys Leu Ala Phe Glu Asn Val Pro Gly Ser Asp Pro His
   225                 230                 235                 240
   Glu Gly Pro Val Gln Ser Ile Cys Thr Val Asp His Ala Glu Arg Ala
                   245                 250                 255
   Phe Ala Glu Tyr Gln Ala Leu Leu Arg Glu Pro Gly Pro Ile Ala Ile
               260                 265                 270
   Gly Ala Met Ala Gly Ala Ser Cys Phe Gly Pro Ala Tyr Glu Tyr Ala
           275                 280                 285
   Met Ile Val Ala Ser Asp Leu Lys Lys Arg Gly Met Glu Gly Pro Val
       290                 295                 300
   Gln Ser Ile Cys Thr Val Asp His Ala Glu Arg Ala Phe Ala Glu Tyr
   305                 310                 315                 320
   Gln Ala Leu Leu Arg Glu Pro Gly Pro Ile Val Ile Gly Ala Met Ala
                   325                 330                 335
   Gly Ala Ser Cys Phe Gly Pro Ala Tyr Glu Tyr Ala Met Ile Val Ala
               340                 345                 350
   Ser Asp Leu Lys Lys Arg Gly Met Arg Asp Lys Ile Pro Ser Phe Thr
           355                 360                 365
   Phe Ile Thr Ser Glu Pro Tyr Leu Gly His Leu Gly Ile Gln Gly Val
       370                 375                 380
   Gly Asp Ser Lys Gly Ile Leu Thr Lys Gly Leu Lys Glu Glu Gly Ile
   385                 390                 395                 400
   Glu Ala Tyr Thr Asn Cys Lys Val Thr Lys Val Glu Asp Asn Lys Met
                   405                 410                 415
   Tyr Val Thr Gln Arg Asp Lys Ile Pro Ser Phe Thr Phe Ile Thr Ser
   420 425 430
   Glu Pro Tyr Ile Gly His Leu Gly Ile Gln Gly Val Gly Asp Ser Lys
   435 440 445
   Gly Ile Leu Thr Lys Gly Leu Lys Glu Glu Gly Ile Glu Ala Tyr Thr
   450 455 460
   Asn Cys Lys Val Thr Lys Val Glu Asp Asn Lys Met Tyr Val Thr Gln
   465 470 475 480
   Val Asp Glu Lys Gly Glu Thr Ile Lys Glu Met Val Leu Pro Val Lys
   485 490 495
   Leu Gly Met Met Ile Pro Ala Phe Lys Gly Val Pro Ala Val Ala Gly
   500 505 510
   Val Glu Gly Leu Cys Asn Pro Gly Gly Phe Val Leu Val Asp Glu His
   515 520 525
   Gln Arg Ser Lys Lys Tyr Ala Asn Ile Phe Ala Ala Val Asp Glu Lys
   530 535 540
   Gly Glu Thr Ile Lys Glu Met Val Leu Pro Val Lys Phe Gly Met Met
   545 550 555 560
   Ile Pro Ala Phe Lys Gly Val Pro Ala Val Ala Gly Val Glu Gly Leu
   565 570 575
   Cys Asn Pro Gly Gly Phe Val Leu Val Asp Glu His Gln Arg Ser Lys
   580 585 590
   Lys Tyr Ala Asn Ile Phe Ala Ala Gly Ile Ala Ile Ala Ile Pro Pro
   595 600 605
   Val Glu Thr Thr Pro Val Pro Thr Gly Ala Pro Lys Thr Gly Tyr Met
   610 615 620
   Ile Glu Ser Met Val Ser Ala Ala Val His Asn Ile Lys Ala Asp Leu
   625 630 635 640
   Glu Gly Arg Lys Gly Glu Gln Thr Met Gly Thr Trp Asn Ala Val Cys
   645 650 655
   Phe Ala Asp Met Gly Ile Ala Ile Ala Ile Pro Pro Val Glu Thr Thr
   660 665 670
   Pro Val Pro Thr Gly Ala Pro Lys Thr Gly Tyr Met Ile Glu Ser Met
           675                 680                 685
   Val Ser Ala Ala Val His Asn Ile Lys Ala Asp Leu Glu Gly Arg Lys
       690                 695                 700
   Gly Glu Gln Thr Met Gly Thr Trp Asn Ala Val Cys Phe Ala Asp Met
   705                 710                 715                 720
   Gly Asp Arg Gly Ala Ala Phe Ile Ala Leu Pro Gln Leu Lys Pro Arg
                   725                 730                 735
   Lys Val Asp Val Phe Ala Tyr Gly Arg Trp Val His Leu Ala Lys Val
               740                 745                 750
   Ala Phe Glu Lys Tyr Phe Ile Arg Lys Met Lys Ile Gly Val Ser Glu
           755                 760                 765
   Pro Phe Tyr Glu Lys Val Leu Phe Lys Met Met Gly Gly Asp Arg Gly
       770                 775                 780
   Ala Ala Phe Ile Ala Leu Pro Gln Leu Lys Pro Arg Lys Val Asp Val
   785                 790                 795                 800
   Phe Ala Tyr Gly Arg Trp Val His Leu Ala Lys Val Ala Phe Glu Lys
                   805                 810                 815
   Tyr Phe Ile Arg Lys Met Lys Met Gly Val Ser Glu Pro Phe Tyr Glu
               820                 825                 830
   Lys Val Leu Phe Lys Met Met Gly Ile Thr Arg Leu Lys Glu Glu Asp
           835                 840                 845
   Ala His Arg Lys Ala Ser Ile Thr Arg Ala Lys Glu Glu Asp Thr His
       850                 855                 860
   Arg Lys Ala Ser
   865